Europäisches Patentamt

European Patent Office

Office européen des brevets

(11) Numéro de publication: **0 377 527**
**A1**

## (12) DEMANDE DE BREVET EUROPEEN

(21) Numéro de dépôt: **90400005.6**

(22) Date de dépôt: **02.01.90**

(51) Int. Cl.⁵: **D01F 4/06, A61K 37/12**

(30) Priorité: **04.01.89 FR 8900056**

(43) Date de publication de la demande:
**11.07.90 Bulletin 90/28**

(84) Etats contractants désignés:
**AT BE CH DE DK ES FR GB GR IT LI LU NL SE**

(71) Demandeur: **Mitz, Vladimir**
**176, boulevard Saint-Germain**
**F-75006 Paris(FR)**

(72) Inventeur: **Mitz, Vladimir**
**176, boulevard Saint-Germain**
**F-75006 Paris(FR)**

(74) Mandataire: **Plaçais, Jean-Yves et al**
**Cabinet Netter, 40, rue Vignon**
**F-75009 Paris(FR)**

(54) **Procédé d'obtention de fibres de collagène de peau d'origine humaine, fibres ainsi obtenues et compositions les contenant.**

(57) L'invention concerne un procédé d'obtention de fibres de collagène obtenues à partir de fragments de peau humaine. On traite le matériau de départ pour obtenir, par action d'une solution de chlorure de magnésium, une suspension de fibrilles de collagène; on reforme in vitro les fibres de collagène sous l'action d'un promoteur de fibrillogénèse constitué par un sel d'acide de la glucosamine et/ou l'acide N-acétyl neuraminique; on contrôle en microscopie les fibres obtenues et, si nécessaire, on les lave par une solution de sorbitan; on rince les fibres et on les utilise dans une composition tamponnée pour des injections dermiques sur le sujet qui a fourni les fragments de peau servant de matériau de départ.

EP 0 377 527 A1

## Procédé d'obtention de fibres de collagène de peau d'origine humaine, fibres ainsi obtenues et composition les contenant

L'invention concerne un procédé d'obtention de fibres de collagène d'origine humaine et l'utilisation de ces fibres pour des traitements thérapeutiques, notamment en chirurgie esthétique.

Il est bien connu que le collagène est une protéine très abondante chez l'homme : on la trouve dans la plupart des tissus et organes et, notamment, dans la peau. Le collagène constitue des structures organisées et, notamment, des fibres ayant une longueur comprise entre 1/10ème de millimètre et quelques millimètres et un diamètre de quelques centaines de nanomètres, lesdites fibres présentant des striations transversales tous les 70 nanomètres environ. Ces fibres de collagène sont constituées par l'association de fibrilles de tropocollagène, ces fibrilles constituant l'élément constitutif de base du matériau collagénique humain.

On a déjà proposé un certain nombre de méthodes permettant, à partir d'un tissu animal, d'extraire de ce tissu, une fraction collagénique par action d'une solution aqueuse d'un sel de métal alcalin ou alcalino-terreux (voir notamment GOLDSTEIN, C.R. ACADEMIE DES SCIENCES DE PARIS, Tome 268, Pages 2446-2448 et la bibliographie citée), mais le collagène ainsi obtenu ne se trouve pas sous forme de fibres organisées, telles qu'on les trouve dans le tissu conjonctif.

Dans les techniques de chirurgie esthétique, il est connu, pour corriger des dépressions dermiques dues par exemple à des séquelles d'acné, à des traumatismes accidentels ou à des séquelles de vieillissement, d'injecter dans les zones dépressionnaires du derme, un matériau collagénique. Jusqu'à présent, le matériau collagénique injecté est généralement d'origine bovine; les injections se font par de fines aiguilles au niveau des fractures du derme. Malheureusement, le collagène bovin ainsi mis en place constitue, par rapport à l'organisme humain dans lequel il est injecté, un corps étranger qui est attaqué par une enzyme, la collagénase, secrétée par les fibroblastes du sujet traité; il en résulte que l'apport collagénique mis en place est résorbé en quelques mois, de sorte que ce type de traitement n'est pas satisfaisant. Pour éviter l'inconvénient précité, il faudrait, d'une part, pouvoir mettre en place dans les fractures dermiques, un collagène de même type (à savoir type I et III) d'origine humaine et il faudrait, d'autre part, que ce collagène soit sous forme de fibres collagéniques pour permettre la reconstitution d'une matrice dermique ayant les mêmes caractéristiques que la matrice collagénique du tissu conjonctif du sujet dans les zones voisines de la zone traitée.

La présente invention a donc essentiellement pour but de proposer un procédé d'obtention de fibres de collagène qui, d'une part, sont d'origine humaine et, d'autre part, ont la forme organisée des fibres de collagène naturel. En d'autres termes, on se propose de prélever un matériau de départ sur un sujet humain, d'en extraire les fibrilles de collagène en les séparant de toutes les parties non collagéniques du tissu environnant, ce qui purifie le matériau de départ, de reconstruire in vitro des fibres de collagène à partir des fibrilles ainsi obtenues et d'utiliser lesdites fibres en suspension pour des injections intradermiques. Cependant, selon une caractéristique complémentaire de l'invention, on propose, pour augmenter la tolérance du sujet receveur vis-à-vis des fibres collagéniques, d'utiliser comme matériau de départ, des fragments de peau du patient receveur : cette technique a, sur le plan de la tolérance, le même avantage que les auto-greffes et, en outre, elle évite tout risque vis-à-vis des maladies transmises par des virus encapsulés du type HIV (SIDA) ou analogues.

Selon l'invention, on a constaté que les fibrilles de collagène que l'on peut obtenir et isoler de façon connue sont susceptibles de se réorganiser en fibres de collagène identiques à celles que l'on trouve dans le tissu conjonctif humain lorsqu'en milieu dilué on les met en présence d'un promoteur de fibrillogénèse à pH faiblement acide. Selon l'invention, on a trouvé que le rendement d'obtention des fibres de collagène reconstituées était suffisant pour donner naissance à un procédé utilisable dans la pratique, lorsque l'on choisissait, comme promoteur de fibrillogénèse, la glucosamine acétylée ou l'acide N-acétyl neuraminique ou leurs mélanges. On obtient ainsi des suspensions de fibres de collagène en milieu aqueux. Pour pouvoir utiliser ces suspensions en chirurgie esthétique, sans pour cela être obligé d'aseptiser, par exemple par irradiation, les compositions obtenues, on mène le procédé d'obtention de façon aseptique de la première à la dernière étape, ce qui est parfaitement suffisant, compte tenu du temps de conservation court, que l'on envisage entre le moment de l'obtention de la suspension injectable et celui de son utilisation sur le sujet receveur, et du caractère autogène du produit.

La présente invention a, en conséquence, pour objet un procédé d'obtention de fibres de collagène de peau d'origine humaine à partir de fibrilles de collagène extraites, de façon connue, de fragments de peau humaine aseptisée en faisant agir, après broyage, une solution aqueuse aseptique d'un sel de métal alcalin ou alcalino-terreux, caractérisé par le fait que, dans un premier temps, on reconstitue les fibres de collagène, après dilution de la suspension de fibrilles, en ajoutant à ladite suspension un promoteur de fibrillogénèse pris dans le groupe formé par la glucosamine acétylée, l'acide N-acétyl neuraminique et leurs mélanges, le pH étant amené à une valeur comprise entre 4 et 6, et en laissant maturer pour assurer la croissance des fibres; que, dans un deuxième temps, on sépare de la suspension-mère, les fibres de collagène obtenues et on les contrôle en microscopie pour vérifier que les fibres de collagène sont exemptes des fractions protéiniques qui leur étaient associées dans le matériau de départ, toute séparation jugée insuffisante donnant lieu, d'abord, à un lavage des fibres par agitation avec une solution aqueuse atoxique d'un détergent non-ionique suivie d'une séparation des fractions protéiniques résiduelles pour obtenir une suspension aqueuse de fibres de collagène exemptes de corps étrangers, puis à au moins un rinçage à l'eau aseptique; et que, dans un troisième temps, on conditionne les fibres pour leur utilisation ultérieure.

Dans un mode préféré de mise en oeuvre du procédé selon l'invention, on réalise l'extraction de fibrilles au moyen d'une solution aqueuse d'un sel de magnésium à un pH basique inférieur à 8; on réalise l'extraction de fibrilles en plusieurs étapes successives, la première phase d'extraction donnant un résidu solide qui est repris dans une phase d'extraction suivante, la concentration molaire en sel de magnésium de la solution aqueuse d'extraction croissant progressivement de 0,1 à 1 M quand on passe de la première à la dernière étape.

Dans le premier temps du procédé selon l'invention, on soumet la suspension de fibrilles à l'action du promoteur de fibrillogénèse; on utilise, de préférence, ce promoteur à une concentration comprise entre 0,005 M et 0,05 M dans la suspension après dilution; on porte tout d'abord la température à une valeur comprise entre 30 et 50°C, de préférence voisine de 37°C, pendant un temps inférieur à 2 heures, après quoi on laisse maturer la suspension pendant un temps compris entre 10 et 50 heures à une température comprise entre 0 et 10°C, de préférence voisine de 4°C. Dans la phase initiale, lorsque la température est élevée, on favorise le début de formation des fibres, alors que dans la deuxième phase, au cours de la maturation, on laisse aux fibres le temps de se développer; il en résulte que les temps ci-dessus indiqués ne sont pas critiques et qu'ils correspondent simplement à des valeurs permettant d'obtenir un développement particulièrement satisfaisant des fibres de collagène. Il est à noter que, pour assurer une action convenable du promoteur de fibrillogénèse, la suspension de fibrilles, telle qu'obtenue dans l'étape d'extraction, doit être diluée; avantageusement, on dilue ladite suspension de 10 à 30 fois par addition d'eau aseptique.

On peut avantageusement prévoir que l'on sépare de la suspension-mère les fibres reconstituées par décantation, en évacuant le surnageant, la partie résiduelle étant reprise par un tamisage dont le filtrat contient les fibres de collagène.

Le contrôle des suspensions de fibres collagéniques en microscopie est généralement effectué au microscope électronique; si l'on constate que des protéines sont collées sur les fibres de collagène, on assure un lavage des fibres au moyen d'une solution de sorbitan (produit commercialisé sous la dénomination "TWEEN 20"); la solution de lavage a une concentration pondérale en sorbitan avantageusement comprise entre 0,5 ‰ et 4 ‰ et, de préférence, voisine de 2 ‰.

La présente invention a également pour objet les fibres de collagène obtenues selon le procédé ci-dessus défini.

L'invention a également pour objet une composition injectable utilisable pour le traitement du corps humain, caractérisée par le fait qu'elle est constituée d'une suspension, dans une phase aqueuse, de fibres de collagène telles que ci-dessus définies, à une concentration comprise entre 1 et 15% en volume et, de préférence, entre 5 et 10% en volume.

La phase aqueuse de la composition selon l'invention est avantageusement un tampon sodique ayant un pH compris entre 7 et 7,5; ce tampon peut être, par exemple, un tampon "PBS" contenant 0,1 M de phosphate monosodique, 0,1 M de phosphate disodique et 0,1 M de chlorure de sodium.

La composition selon l'invention peut également contenir des substances à activité thérapeutique; ces substances peuvent être, par exemple, des hormones, des antibiotiques, ou des antimitotiques.

Dans la composition selon l'invention, les fibres de collagène peuvent avantageusement être obtenues à partir d'un matériau de départ prélevé sur le sujet qui doit être traité par ladite composition.

Pour mieux faire comprendre l'objet de l'invention, on va en décrire maintenant, à titre d'exemple purement illustratif et non limitatif, un mode de mise en oeuvre.

Dans cet exemple, on va décrire la préparation d'une composition destinée à être utilisée en injection dermique dans les fractures du derme d'un patient présentant des rides dues au vieillissement.

On prélève sur le patient, par une première opération de chirurgie esthétique, notamment un lifting ou une plastie mammaire, fessière ou abdominale, un certain nombre de grammes de peau, cette peau comportant l'épiderme et 3 à 4 mm du derme sous-jacent. La peau du sujet a initialement été aseptisée par le chirurgien de façon connue. Si l'on suppose une opération de lifting, on prélève par exemple 20 g de peau et on dissèque cette peau pour en éliminer éventuellement la graisse. On obtient ainsi 10 g de matériau de départ dégraissé et aseptique.

On broie immédiatement ce matériau dans une solution aqueuse aseptique de chlorure de magnésium tamponné à pH 7,5 par un tampon (acide citrique/tris). On utilise trois volumes de solution saline pour un volume de peau préparée. Le broyage dans la solution saline est effectué à l'aide d'un broyeur à couteau pendant 30 minutes à 4°C. La suspension obtenue est agitée pendant 12 heures et maintenue à 4°C. On passe alors le mélange sur un tamis ayant une ouverture de maille de 0,25 mm et l'on sépare ainsi la phase liquide du résidu solide. Dans cette première phase d'extraction, la solution saline a une concentration 0,10 M. Le résidu solide séparé est repris et soumis à une nouvelle phase d'extraction avec une solution de chlorure de magnésium 0,3 M. Le même processus d'extraction est appliqué et permet ainsi d'obtenir une nouvelle fraction liquide. On reprend à nouveau le résidu solide et l'on effectue ainsi cinq extractions successives, la concentration en chlorure de magnésium de la solution saline utilisée croissant d'extraction en extraction jusqu'à atteindre 1 M pour la dernière extraction. Les cinq fractions liquides obtenues sont mélangées et constituent la suspension de fibrilles à laquelle on va faire subir l'étape de fibrillogénèse.

On dilue la suspension de fibrilles obtenue en lui ajoutant 20 fois son volume d'eau aseptique et on ajoute, dans cette suspension maintenue sous faible agitation, de l'acide N-acétyl neuraminique pour obtenir une concentration de 0,02 M. On arrête l'agitation et on amène la suspension à 37°C pendant 30 minutes; puis on fait baisser la température à 4°C et on la maintient à cette valeur pendant 16 heures. Une fraction solide décante lentement au fond du récipient : on extrait le surnageant qui contient des fractions protéiniques hydrosolubles. On reprend le résidu solide sur un tamis ayant une ouverture de maille de 0,1 mm pour séparer les résidus tissulaires : le filtrat est une suspension de fibres de collagène.

On contrôle la suspension ainsi obtenue au microscope électronique. Si l'on constate que des éléments protéiniques sont collés sur les fibres de collagène, on effectue un lavage des fibres au moyen d'un détergent. Pour ce faire, on ajoute dans la suspension de fibres 2 ‰ de sorbitan vendu sous la dénomination commerciale "TWEEN 20" et on maintient le mélange sous agitation pendant 12 heures. On laisse ensuite décanter, on extrait le surnageant et on rince à l'eau, l'opération de décantation et de rinçage étant recommencée dix fois de suite pour assurer l'extraction totale du détergent. On contrôle éventuellement ensuite au microscope électronique que les fibres de collagène obtenues sont parfaitement propres.

La totalité des opérations ci-dessus décrites est réalisée dans des conditions de stérilité chirurgicale, sans addition d'aucun antiseptique et sans irradiation.

La suspension obtenue est utilisée pour constituer une composition injectable contenant 7% en volume de fibres en présence d'un tampon sodique PBS qui ajuste le pH à une valeur de 7,3. Ce tampon répond à la formulation suivante :
0,1 M de phosphate monosodique
0,1 M de phosphate disodique
0,10 M de chlorure de sodium.

Cette composition, stockée à 4°C, est injectée, quelques jours après sa préparation, dans les fractures dermiques des rides du sujet sur lequel a été effectué le prélèvement de la peau qui a servi de matière première de départ.

On constate que la mise en place de ces fibres de collagène dans lesdites fractures dermiques permet d'obtenir un excellent résultat, sans aucune manifestation allergique; le résultat est, en outre, maintenu au cours du temps, ce qui est un avantage appréciable par rapport à l'utilisation antérieure de collagène bovin, dont l'effet disparaissait au bout de quelques mois.

**Revendications**

1.- Procédé d'obtention de fibres de collagène de peau d'origine humaine à partir de fibrilles de collagène extraites, de façon connue, de fragments de peau humaine aseptisée en faisant agir, après broyage, une solution aqueuse aseptique d'un sel de métal alcalin ou alcalino-terreux, caractérisé par le fait que, dans un premier temps, on reconstitue les fibres de collagène, après dilution de la suspension de fibrilles, en ajoutant à ladite suspension un promoteur de fibrillogénèse pris dans un groupe formé par la glucosamine acétylée, l'acide N-acétyl neuraminique et leurs mélanges, le pH étant amené à une valeur comprise entre 4 et 6, et en laissant maturer pour assurer la croissance des fibres; que, dans un deuxième temps, on sépare de la suspension-mère, les fibres de collagène obtenues et on les contrôle en microscopie pour vérifier que les fibres de collagène sont exemptes des fractions protéiniques qui leur étaient associées dans le matériau de départ, toute séparation jugée insuffisante donnant lieu, d'abord, à un lavage des fibres par agitation avec une solution aqueuse atoxique d'un détergent non-ionique suivie d'une séparation des fractions protéiniques résiduelles pour obtenir une suspension aqueuse de fibres de collagène exemptes de corps étranger, puis à au moins un rinçage à l'eau aseptique; et que, dans un troisième temps, on conditionne les fibres pour leur utilisation ultérieure.

2.- Procédé selon la revendication 1, caractérisé par le fait que l'on réalise l'extraction de fibrilles au moyen d'une solution aqueuse d'un sel de magnésium à un pH basique inférieur à 8.

3.- Procédé selon la revendication 2, caractérisé par le fait que l'on réalise l'extraction de fibrilles en plusieurs étapes successives, la première phase d'extraction donnant un résidu solide qui est repris dans une phase d'extraction suivante, la concentration molaire en sel de magnésium de la solution aqueuse d'extraction croissant progressivement de 0,1 à 1 M quand on passe de la première à la dernière étape.

4.- Procédé selon l'une des revendications 1 à 3, caractérisé par le fait que, lorsque le contrôle des fibres en microscopie l'exige, on lave lesdites fibres au moyen d'une solution de sorbitan.

5.- Procédé selon la revendication 4, caractérisé par le fait que la solution de lavage a une concentration pondérale en sorbitan comprise entre 0,5 ‰ et 4 ‰.

6.- Procédé selon l'une des revendications 1 à 5, caractérisé par le fait que l'on utilise un promoteur de fibrillogénèse à une concentration comprise entre 0,005 M et 0,05 M dans la suspension après dilution.

7.- Procédé selon l'une des revendications 1 à 6, caractérisé par le fait que l'on réalise la reconstitution des fibres de collagène en portant la température entre 30 et 50°C pendant un temps inférieur à 2 heures après l'introduction du promoteur de fibrillogénèse et en laissant ensuite maturer entre 0 et 10°C pendant un temps compris entre 10 et 50 heures.

8.- Procédé selon l'une des revendications 1 à 7, caractérisé par le fait qu'avant d'ajouter le promoteur de fibrillogénèse, on dilue la suspension de 10 à 30 fois par addition d'eau aseptique.

9.- Procédé selon l'une des revendications 1 à 8, caractérisé par le fait que l'on sépare de la suspension-mère les fibres reconstituées par décantation, en évacuant le surnageant, la partie résiduelle étant reprise par un tamisage, dont le filtrat contient les fibres.

10.- Fibres de collagène obtenues selon le procédé de l'une des revendications 1 à 9.

11.- Composition injectable utilisable pour un traitement du corps humain, caractérisée par le fait qu'elle est constituée d'une suspension, dans une phase aqueuse, de fibres selon la revendication 10 à une concentration comprise entre 1 et 15% en volume et, de préférence, entre 5 et 10% en volume.

12.- Composition selon la revendication 11, caractérisée par le fait que la phase aqueuse est un tampon sodique ayant un pH compris entre 7 et 7,5.

13.- Composition selon la revendication 12, caractérisée par le fait que le tampon contient 0,1 M de phosphate monosodique, 0,1 M de phosphate disodique et 0,1 M de chlorure de sodium.

14.- Composition selon l'une des revendications 11 à 13, caractérisée par le fait qu'elle contient des substances à activité thérapeutique.

15.- Composition selon la revendication 14, caractérisée par le fait que les substances à activité thérapeutique sont prises dans le groupe formé par les hormones, les antibiotiques et les antimitotiques.

16.- Composition selon l'une des revendications 11 à 15, caractérisée par le fait que les fibres de collagène qu'elle contient sont obtenues à partir d'un matériau de départ prélevé sur le sujet, qui doit être traité par ladite composition.

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | US-A-3 949 073 (J.R. DANIELS et al.) <br> * Revendications; colonne 2, lignes 31-33,52 * <br> --- | 1,11 | D 01 F 4/06 <br> A 61 K 37/12 |
| A | EP-A-0 089 145 (COLLAGEN CORP.) <br> * Page 4, lignes 4-6 * <br> --- | 1 | |
| A | EP-A-0 196 197 (COLLAGEN CORP.) <br> --- | | |
| A | FR-A-2 328 786 (COLLAGEN CORP.) <br> ----- | | |

DOMAINES TECHNIQUES RECHERCHES (Int. Cl.5)

D 01 F
A 61 K

Le présent rapport a été établi pour toutes les revendications

| Lieu de la recherche | Date d'achèvement de la recherche | Examinateur |
|---|---|---|
| LA HAYE | 19-04-1990 | VAN GOETHEM G.A.J.M. |